**Europäisches Patentamt**

**European Patent Office**

**Office européen des brevets**

(19)

(11) Veröffentlichungsnummer: **0 282 645**
**B1**

(12)

# EUROPÄISCHE PATENTSCHRIFT

(45) Veröffentlichungstag der Patentschrift:
**30.05.90**

(51) Int. Cl.⁵: **A61F 5/11**

(21) Anmeldenummer: **87117442.1**

(22) Anmeldetag: **26.11.87**

(54) Vorrichtung zur Vornahme von Nagelkorrekturen.

(30) Priorität: **18.03.87 DE 3708811**

(43) Veröffentlichungstag der Anmeldung:
**21.09.88 Patentblatt 88/38**

(45) Bekanntmachung des Hinweises auf die Patenterteilung:
**30.05.90 Patentblatt 90/22**

(84) Benannte Vertragsstaaten:
**AT BE CH DE ES FR GB IT LI NL SE**

(56) Entgegenhaltungen:
**DE-A- 3 330 813**
**US-A- 2 505 086**
**US-A- 4 157 095**
**US-A- 4 674 486**

(73) Patentinhaber: **Stolz, Bernd, Hopfengartenstrasse 26,**
**D-8451 Ammerthal(DE)**

(72) Erfinder: **Stolz, Bernd, Hopfengartenstrasse 26,**
**D-8451 Ammerthal(DE)**

(74) Vertreter: **Schneck, Herbert, Dipl.-Phys., Dr. et al, Rau &**
**Schneck Patentanwälte Königstrasse 2,**
**D-8500 Nürnberg 1(DE)**

ACTORUM AG

**Beschreibung**

Die Erfindung richtet sich auf eine Vorrichtung zur Vornahme von Nagelkorrekturen unter Verwendung eines blattfederartigen Streifens, insbesondere bei eingewachsenen, zu stark gekrümmten Nägeln.

Eine derartige Vorrichtung ist aus der DE-A 3 330 813 bekannt. Danach ist vorgesehen, daß ein blattfederartiger Streifen dazu verwendet wird, um einen zu stark gekrümmten Nagel seitlich anzuheben, wobei im Bereich der seitlichen Außenränder des Nagels bügelförmige Befestigungseinrichtungen angeklebt werden und die metallische Blattfeder dann in Ausnehmungen der bügelförmigen Befestigungsteile eingeschoben wird.

Hierdurch wird gegenüber herkömmlichen Verfahren bereits ein relativ guter Tragekomfort bei sehr guter kosmetischer Korrekturwirkung erreicht.

Allerdings umfaßt die vorbekannte Vorrichtung bzw. das entsprechende Befestigungsverfahren insgesamt drei relativ kleine Einzelteile, nämlich das Federelement und die beiden Befestigungsteile, welche zu ihrer Handhabung eines gewissen Geschickes des jeweiligen Fußpflegers bedürfen und welche zudem auch einen gewissen Herstellungsaufwand brauchen.

Hiervon ausgehend liegt der Erfindung die Aufgabe zugrunde, eine Vorrichtung der eingangs genannten Art so auszugestalten, daß der angestrebte Justiereffekt möglichst einfach und kostengünstig erreicht wird.

Diese Aufgabe wird gelöst durch eine solche Vorrichtung, welche sich dadurch auszeichnet, daß der Streifen aus elastischem Kunststoff vorzugsweise einem glasfaserverstärkten Duroplast besteht.

Versuche haben gezeigt, daß sich zwischen Kunststoff und menschlichen Nägeln mittels Schnellkleber eine sehr schnell wirksame und außerordentlich haltbare Verbindung herstellen läßt. Dementsprechend kann der Kunststoff-Streifen in ca. 6 Sekunden längs seiner ganzen Länge an den zu korrigierenden Nagel angeklebt werden, ohne daß gesonderte Befestigungsteile vorgesehen werden müßten, was darüber hinaus noch den wesentlichen Vorteil bringt, daß die Kraftübertragung der Rückstellkraft des blattfederartigen Streifens nicht nur über die Klebeflächen der Befestigungsteile sondern längs des gesamten Streifens gleichmäßig und damit weitestgehend schmerzfrei erfolgt.

Günstigerweise ist vorgesehen, daß die Nagelplatte vor der Anbringung des elastischen Streifens durch Abschleifen und Entfetten vorbereitet wird.

Vorteilhafterweise wird der Streifen zunächst in der Mitte, dann an der einen Außenseite und dann an der anderen Außenseite verklebt. Dies bedeutet, daß zunächst in der Mitte eine örtlich begrenzte Klebeverbindung hergestellt wird, deren Aushärten abgewartet wird, und daß erst dann die seitlichen Randbereiche befestigt werden. Hierdurch wird die angestrebte gleichmäßige Kraftverteilung derart erzielt, daß insbesondere an den Rändern die gewünschten Korrekturkräfte auftreten, wobei gleichzeitig eine exakte Justierung des Streifens möglich ist.

Ein ganz besonderer Vorteil der erfindungsgemäßen Vorrichtung liegt darin, daß eine sehr exakte, individuelle Korrektur der Rückstellkraft des Streifens möglich ist, wenn sich herausstellt, daß nach dessen Befestigung ein Schmerz festgestellt wird. Es kann dann nämlich durch einfaches Abschleifen des Streifens dessen Federkraft reduziert werden, wobei es auf diese Weise vor allem auch möglich ist, die Federkraft z.B. nur auf einer, gegebenenfalls schmerzenden Nagelseite zu reduzieren und diese andererseits auf der gegenüberliegenden Seite voll wirksam werden zu lassen. Eine derartige individuelle Korrekturmöglichkeit besteht bei der Verwendung von Herkömmlichen metallischen Federelementen, welche in Befestigungsteilen festgelegt werden, nicht.

Zur Erzielung einer besonders haltbaren Verbindung, welche darüber hinaus gegenüber Strümpfen oder Schuhen keine freien Stoßkanten entstehen läßt, ist vorgesehen, daß der Streifen nach der Befestigung versiegelt wird, wobei hierfür im einfachsten Fall der zur Befestigung verwendete Schnellkleber herangezogen wird. Ein derart befestigter und versiegelter Streifen ist, insbesondere bei einer farblichen Anpassung des Streifens an die natürliche Nagelfarbe, äußerst unauffällig und erfüllt damit hohe kosmetische Ansprüche, zumal es möglich ist, den gesamten Nagel mit daran befestigtem Streifen problemlos zu lackieren.

Nachfolgend wird die Anwendung der erfindungsgemäßen Vorrichtung anhand eines Ausführungsbeispiels beschrieben:

Korrigiert werden soll ein Fußnagel, der insbesondere im Bereich seiner seitlichen Außenränder zu stark gekrümmt ist und dementsprechend nicht nur unschön anzusehen, sondern auch schmerzverursachend ist.

Der Nagel wird zunächst so vorbereitet, daß ein sich quer zwischen den seitlichen Außenrändern erstreckender Bereich mittels einer üblichen Schleifvorrichtung aufgerauht und mit Hilfe eines Entfettungsmittels entfettet wird.

Aus einer Mehrzahl von Kunststoffstreifen unterschiedlicher Länge wird ein solcher ausgewählt, welcher vom seitlichen Nagelwall z.B. einen Abstand von ca. 1 mm aufweist.

Der Kunststoffstreifen besteht aus einem glasfaserverstärkten Duroplast, z.B. HGW 2372.4. Der Streifen ist durch Ausstanzen aus einem entsprechenden Plattenmaterial hergestellt und weist zur Vermeidung von Druckpunkten abgerundete Enden auf. Der Streifen ist weniger als 0,5 mm dick.

Zur Anbringung des Streifens wird am einfachsten die Nagelmitte und die Streifenmitte markiert und der Streifen dann zunächst entsprechend dieser Markierung mit einem Schnellkleber fixiert. Der Schnellkleber härtet nach ca. 6 Sekunden aus, so daß dann der Streifen in der Mitte festliegt, die beiden äußeren Enden sind noch lose. Es wird dann anschließend Kleber zunächst auf die eine Unterseite des Streifens gebracht und dieser angedrückt, bis er fixiert ist. Anschließend wird mit der noch freien Seite in gleicher Weise verfahren.

Um ein definiertes Positionieren und Fixieren des Streifens zu ermöglichen, eignet sich besonders gut ein spezielles Werkzeug, welches in etwa die Form eines Bleistiftes aufweist, dessen Spitze mit einer sich nach innen erstreckenden, konkaven Vertiefung versehen ist. Hierdurch wird eine besonders gute Griffigkeit im Kantenbereich des Streifens erreicht.

Das andere Ende dieses Werkzeugs kann in Form eines sehr kleinen Spatels ausgebildet sein und dementsprechend zum Auftragen des Klebstoffs verwendet werden.

Sollte sich nach der Fixierung des Streifens die Korrekturkraft desselben als zu stark erweisen und ein Schmerz festgestellt werden, kann mittels eines Korundschleifsteins der Streifen dünner geschliffen werden, um so die Federkraft zu reduzieren.

Abschließend wird eine Versiegelung mit Hilfe des verwendeten Klebers vorgenommen, insbesondere im Bereich der Randkanten. Die Versiegelung trocknet nach ca. 3 Minuten aus. Der fertig fixierte Streifen steht über den Nagel dann kaum wesentlich hinaus und stellt damit für den Träger keine wesentliche Behinderung dar.

Sollte sich bei besonders starken oder besonders stark gekrümmten Nägeln eine höhere Korrekturkraft als erforderlich erweisen, können ohne weiteres auch zwei derartige Streifen parallel zueinander angeordnet werden. Es besteht also die Möglichkeit einer sehr individuellen Einstellung der Korrekturkraft.

## Patentansprüche

1. Vorrichtung zur Vornahme von Nagelkorrekturen in Form eines blattfederartigen Streifens, dadurch gekennzeichnet, daß der Streifen aus elastischem Kunststoff besteht.

2. Vorrichtung nach Anspruch 1, dadurch gekennzeichnet, daß der Streifen aus einem glasfaserverstärkten Duroplast besteht.

## Claims

1. Device for carrying out nail corrections in form of a leaf spring type strip, characterized in that the strip consists of elastic plastic material.

2. Device according to claim 1, characterized in that the strip consists of a glass fibre reinforced duroplast.

## Revendications

1. Dispositif pour effectuer des corrections d'ongles, se présentant sous la forme d'une bande du type lamelle de ressort, caractérisé en ce que la bande est constituée d'une matière plastique élastique.

2. Dispositif selon la revendication 1, caractérisé en ce que la bande est constituée d'une résine thermodurcissable armée de fibres de verre.